# EUROPEAN PATENT SPECIFICATION

(11) **EP 1 282 393 B1**
(45) Date of publication and mention of the grant of the patent: **09.11.2005**
(21) Application number: 01932343.5
(22) Date of filing: 16.05.2001
(51) Int. Cl.: A61K 7/40, A61K 7/48, A61K 9/00

(54) **EXTERNAL APPLICATION FOR ENHANCING THE SKIN PERMEABILITY OF THE ACTIVE COMPONENTS THEREIN**
ZUBEREITUNG ZUR ÄUSSERLICHEN ANWENDUNG ZUR VERBESSERUNG DER PERMEABILITÄT DER HAUT FÜR WIRKSTOFFE
COMPOSITION D'APPLICATION EXTERNE AMELIORANT LA PERMEABILITE DE LA PEAU AUX INGREDIENTS ACTIFS DE LA COMPOSITION

(30) Priority: 16.05.2000 KR 2000026086
(43) Date of publication of application: 12.02.2003
(73) Proprietor: PACIFIC CORPORATION, Seoul 140-777 (KR)
(72) Inventor: SHIN, Eui Seok, Youngin-shi Kyungki-do 449-900 (KR); LEE, Jang Young, Seoul 151-019 (KR); KIM, Mu Sung, Suwon-shi Kyungki-do 442-190 (KR); LEE, Sung Gu, Paldal Suwon-shi Kyungki 442-470 (KR); LEE, Dong Chul, Sungnam-shi Kyunggi-do 463-070 (KR); KANG, Byung Young, Dongjak-ku Seoul 156-010 (KR)
(74) Representative: Audier, Philippe André
(86) International application number: PCT/KR2001/000788
(87) International publication number: WO 2001/087255

(56) References cited:
- WO-A2-99/15210
- FR-A- 2 791 059
- JP-A- 3 209 333
- KR-A- 2000 060 771
- SIM, LEE ET AL.: "Stabilization of papain and lysozyme for application to cosmetic products" BIOTECHNOLOGY LETTERS, vol. 22, 2000, pages 137-140, XP009025462

## Description

### FIELD OF THE INVENTION

The present invention relates to the non-therapeutic use of a protease as a skin absorption agent of an active agent of a cosmetic composition.

### BACKGROUND OF THE INVENTION

Skin is divided into three portions, a stratum corneum, epidermal layer and dermis, and the most important barrier for skin absorption of the active agent contained in the external application such as cosmetics is the stratum corneum located on the outermost portion.

Espacially, because the stratum corneum is composed of corneous cell mainly comprising keratins and lipid layer filling the space between corneous cells, liposoluble active agents are well absorbed into the skin while water-soluble agents and large-molecule agents are not Therefore, most of the agents infused in the external applications such as cosmetics are not easily absorbed into the skin, because they are water-soluble.

Conventionally, to solve these problems, using non-polar solvents, surfactants, and lipid acid, et al. as skin absorption enhancers is disclosed. [Chen LH, Chien YW, "Enhancement of skin penetration" In "Novel cosmetic delivery systems" 1990, 60; Rhein LD, Robbins CR, Fernee K, Cantore R "Surfactant structure effects on swelling of isolated human stratum corneum", J. Soc. Cosmet Chem. 1986, 37: 125; Cooper ER, Merritt EW, Smith RL., "Effect of fatty acids and alcohols on the penetration of acylclovir across human skin in vitro," J. Pharm. Sci. 1985; 74: 688]. However, although these skin absorption enhancers enhance the skin-absorption of the active agent, they cause fierce skin stimuli after being absorbed into the skin because their molecular weights are small.

Further, applying sonophoresis or minute iontophoresis on the skin to enhance the skin absorption of the water-soluble materials to the skin is also disclosed. [Sloan JB, Slotani K. "Iontophoresis in dermatology: a review" J. Am. Acad. Dermatol. 1986, 4:671; Langer R. "Ultrasound-mediated transdermal protein delivery" Science 1995; 269:850] However, because these methods need expensive equipments, these methods are not practically used in the external applications such as cosmetic compositions.

In addition, some patents teach other methods for enhancing the skin absorption of the active agents by modifying or weakening the skin barrier function with enzyme. For example, U.S. Patent Nos. 5,534,260 and 5,296,222 show methods for enhancing the absorption of the active agents by adding protease. However, the above methods are just adding protease into the composition, and therefore, the protease is degenerated or loses its activity with time by the effects of other elements infused in the formulation. As can be estimated above, because the enzyme (active agent) does not accomplish enhanced skin absorption, the above methods are not suitable for external application comprising a lot of elements in addition to the active agent.

Documents FR-A-2 791 059 (Pacific GRP) and SIM, LEE et al. : « Stabilization of papein and hydrozyne for application to cosmetic products » Biotechnology Letters, vol. 22, 2000, pages 137-140 discloses only the stabilized protease of the present invention. However, they do not disclose or suggest that the stabilized protease is useful for enhancing the skin absorption property of the active agent in a cosmetic composition.

To solve above-mentioned problems, inventors of the present invention searched for a method for enhancing the skin absorption of the active agents infused in the external application such as cosmetics, and finally found that when protease stabilized by β-1,3-glucan branched with β-1,6-linkage is used as an agent for enhancing the skin absorption in the external application, the absorption rate is increased.

### SUMMARY OF THE INVENTION

Therefore, an object of the present invention is to provide a composition for external application to the skin having the properties of enhanced skin absorbency of the active agents infused in the composition.

### DETAILED DESCRIPTION OF THE INVENTION

Hereinafter, the present invention is described in detail.

The present invention provides a composition for external skin application with enhanced skin absorption properties by using protease stabilized by β-1,3-glucan branched with β-1,6-linkage as skin absorption enhancing agent in the skin external application. Said composition enhances the skin absorption by modifying the structure of the corneous layer or removing the layer without causing skin stimulus.

A method for preparing the protease used as a skin absorption enhancing agent of the present composition complies with the method described in KR 2000-60771 A1(published on Oct. 16, 2000) entitled "Method for preparing stabilized enzyme or protein, and composition for external application comprising the stabilized enzyme or protein provided by the method". In detail, the method for stabilizing the protease comprises the steps of; (1) reacting a glucan with periodates to transform the β-1,6-linkage of the glucan into aldehyde; (2) removing unreacted periodates from the reaction solution of step (1); (3) adding a protease to the reaction solution of step (2) in an amount of 0.00001 ~ 10.0% by weight; (4) adding a reducing agent to the reaction solution of step (3) in an amount of 0.0001 ~ 1.0% by weight; and (5) washing the product of step (4).

Glucans used in the above method are β-1,3-glucans branched with β-1,6-linkage, and the glucans can make functional groups only in the β-1,6-linkage depending on the chemical reaction. Glucans used in the present invention are not restricted, preferably schizophllan derived from Schizopyllum commune, scleroglucan derived from Sclerotinia sp. and lentinan derived from Lentinus edodes are used.

In addition, proteases stabilized by the method of the present invention are not restricted, preferably papain, chymotrypsin, trypsin, carboxypepidase, pepsin, et al. are used.

The amount of the stabilized protease contained in the composition of the external application is 0.0001 ∼ 99.9999 % by weight on the basis of the weight of total composition. Further, formulation for external application is not restricted. For example, they may have cosmetic formulation such as skin softener, nutrition water, massage, cream, make-up base, lipstick, pack, gel, shampoo, rinse, hair tonic or soap, or external dermatological formulation such as lotion, ointment, gel, cream, patch or spray.

In addition, the sorts of active agents having their skin absorption properties enhanced by the skin absorption enhancers of the present invention are conventional active agents, and are not restricted specifically. For example, water-soluble derivatives of L-ascorbic acid such as L-ascorbic acid, L-ascorbic phosphate, L-ascorbic glucoside, et aL, vitamin B, kojic acid, kojic caffeine acid, kojic aminopropyl phosphate, hydroquinone, arbutin, water soluble natural extract derived from green tea or grape by water or ethanol, et al. are used as active agents.

Hereinafter, the present invention is described more specifically with the embodiments of the present invention, however, the scope of the present invention is not restricted within the embodiments.

### DESCRIPTION OF THE PREFERRED EMBODIMENTS

### <Reference Example 1> Preparation of stabilized Papain

1) To 50 ml of 0.5wt% aqueous solution of sschizophyllan (molecular weight of 2,000,000), 2g of sodium iodide peroxide (NaIO₄) was added, and the resulting mixture was kept in the dark at 4 °C for 1 hour with stirring.
2) The resulting schizophyllan solution was subjected to dialysis over membrane (MW cut off 10,000) in the dark for 48 hours. The dialysis was carried out with replacements of 2500ml water every 12 hours.
3) When the volume of the schizophyllan solution was increased to about 100ml, 0.1wt% of papain was added, and the resulting solution was stirred for 2 hours in the dark at 4°C.
4) 0.05g of sodium borohydride (NaBH₄) was added to the schizophyllan -papain solution, and followed by stirring for 4 hours. Then, 0.1g of lysine was added and the resulting mixture was stirred for 2 hours in the dark at 4°C.
5) The resulting schizophyllan-papain solution was subjected to the dialysis as described in the step 2) to obtain a solution of papain coupled to schizophyllan (GE-1). The yield of the GE-1 prepared was 95% based on the enzyme activity.

### <Example 1 and Comparative Example 1 and 2> Water-gel type cosmestics

| Ingredients | Example 1 | Comparative Examle 1 | Comparative Example 2 |
|---|---|---|---|
| 1. glycerin | 7.0 | 7.0 | 7.0 |
| 2. Carboxyvinyl Polymer | 0.5 | 0.5 | 0.5 |
| 3. Butylene Glycol | 3.0 | 3.0 | 3.0 |
| 4. EDTA-Na₂ | 0.2 | 0.2 | 0.2 |
| 5. Stabilized Papain (Reference Example 1, GE-1) | 1.0 | - | - |
| 6. Papain | - | - | 1.0 |
| 7. Kojic acid | 0.5 | 0.5 | 0.5 |
| 8. Calcium hydroxide | 0.05 | 0.05 | 0.05 |
| 9. Ethanol | 5.0 | 5.0 | 5.0 |
| 10. Preservatives | q.s. | q.s. | q.s. |
| 11. PEG-60 hardened caster oil | 0.3 | 0.3 | 0.3 |
| 12. Perfumes | q.s. | q.s. | q.s. |
| 13. Distilled water | To 100 | To 100 | To 100 |

### <Method for preparation>

1) Ingredients 1~7 were added to the distilled water, then completely dissolved by stirring.
2) Ingredients 10 ~ 12 were added to the ethanol, then completely dissolved by stirring.
3) Solution prepared in step 2) was slowly added to the solution prepared in step 1) while stirring the solution.
4) Ingredient 8 was added to the solution of step 3) to form a gel.
5) Gas bubbles were removed by vacuum deaeration, then a water-gel type cosmestic was obtained.

### <Example 2 and Comparative Example 3 and 4> Water-in-Oil type emulsion cosmestics

| Ingredients | Example 2 | Comparative Example 3 | Comparative Example 4 |
|---|---|---|---|
| 1. Stearic acid | 1.0 | 1.0 | 1.0 |
| 2. Cetostearyl alcohol | 0.7 | 0.7 | 0.7 |
| 3. Microcrystalline wax | 0.2 | 0.2 | 0.2 |
| 4. Monostearic acid glycerin | 0.5 | 0.5 | 0.5 |
| 5. Fluid paraffin | 5.0 | 5.0 | 5.0 |
| 6. Monostearic acid sorbitan | 0.3 | 0.3 | 0.3 |
| 7. Squalane | 3.5 | 3.5 | 3.5 |
| 8. Distilled water | To 100 | To 100 | To 100 |
| 9. Dense glycerin | 6.5 | 6.5 | 6.5 |
| 10. Hyaluronic acid extract | 0.5 | 0.5 | 0.5 |
| 11. Sepigel 305 | 0.7 | 0.7 | 0.7 |
| 12. Stabilized Papain (Reference Example 1, GE-1) | 1.0 | - | - |
| 13. Papain | - | - | 1.0 |
| 14. Ascorbic acid | 0.5 | 0.5 | 0.5 |
| 15. Perfumes | q.s. | q.s. | q.s. |
| 16. Preservatives | q.s. | q.s. | q.s. |

### <Method for preparation>

1) Component A (ingredient 1∼7) and Component B (ingredient 8~10) were heated to 75 °C.
2) A was slowly added to B while stirring at 7,000 rpm. After completely adding A to B, ingredients 14 and 16 were added to the mixture immediately, then stirred for two minute to emulsify.
3) Ingredients 11 and 15 were added and stirred for two minute to emulsify.
4) Mixture of 3) was stirred for 1 minute at 2,500 rpm to remove air.
5) Mixture of 4) was cooled in an ice bath to 28~30°C.
6) Ingredient 12 or 13 was added to the above cooled mixture, then stirred with 2,000 rpm.
7) The product was left to stand at room temperature for 24 hours to obtain a stabilized water-in-oil type cosmetic composition.

### <Experimental Example 1> Measurement for skin absorption of the kojic acid

Measurement for skin absorption of the kojic acid was practiced in the Frantz permeation cells using the skins of a guinea pig. Abdominal skin was obtained from the guinea pig before the experiment. Above obtained skin was cut to a dimension of 1cm², and stood in the permeation cell having the diameter of the permeation lens 0.9cm, then held by the clamp. 0.5 ml of cosmetic compositions from Example 1 and Comparative Example 1 to be tested were applied on one side of the skin, while the other side of the skin was contacted with solvent mixture of distilled water and glycerin with the ratio of 1:1. The temperature was maintained at 32°C, which is the skin temperature. The solvent was collected periodically (with fixed time interval; hour), then the amount of the kojic acid absorbed into the skin was measured by HPLC. The results are shown in Table 1.

**<Table 1>**

| Skin absorption of the kojic acid per applying density(µm/cm²/wt%) | | |
|---|---|---|
| Time(hour) | Example 1 | Comparative Example 1 |
| 0 | 0 | 0 |
| 4 | 6.15 | 1.42 |
| 8 | 12.98 | 3.09 |
| 24 | 36.13 | 9.11 |

Considering that the duration time of general make-up is 4~8 hours, table 1 shows that when a stabilized protease is added to the composition, the skin absorption of kojic acid is increased by about four times compared with a case that a stabilized protease is not added.

### <Experimental Example 2> Measurement for skin absorption of the ascorbic acid

The amount of the ascorbic acid absorbed into the skin was measured with the method described in Experimental Example 1 except that water-in-oil type cosmetic composition of Example 2 and Comparative Example 3 were used. The results are shown in Table 2.

**<Table 2>**

| Skin absorption of the ascorbic acid (µm/cm²/wt%) | | |
|---|---|---|
| Time(hour) | Example 2 | Comparative Example 3 |
| 0 | 0 | 0 |
| 4 | 3.67 | 0.67 |
| 8 | 7.05 | 1.29 |
| 24 | 20.93 | 4.47 |

Table 2 shows that when a stabilized protease is added to the composition, the skin absorption of ascorbic acid is increased by about six times compared with a case that a stabilized protease is not added.

| <Example 3 and Comparative Example 5 and 6> Ointment | | | |
|---|---|---|---|
| Ingredient | Example 3 | Comparative Example 5 | Comparative Example 6 |
| 1. Bee wax | 10.0 | 10.0 | 10.0 |
| 2. Polysorbate 60 | 5.0 | 5.0 | 5.0 |
| 3. PGE-60 hardened castor oil | 2.0 | 2.0 | 2.0 |
| 4. Sorbitan Cesquioleate | 0.5 | 0.5 | 0.5 |
| 5. Vaseline | 5.0 | 5.0 | 5.0 |
| 6. Fluid Paraffin | 10.0 | 10.0 | 10.0 |
| 7. Squalane | 5.0 | 5.0 | 5.0 |
| 8. Shear butter | 3.0 | 3.0 | 3.0 |
| 9. Carprylic/Capric triglyceride | 5.0 | 5.0 | 5.0 |
| 10. Glycerin | 10.0 | 10.0 | 10.0 |
| 11. Propylene glycol | 5.0 | 5.0 | 5.0 |
| 12. Triethanolamine | 0.2 | 0.2 | 0.2 |
| 13. Perfumes | s.q. | s.q. | s.q. |
| 14. Stabilized Papain (Reference Example 1, GE-1) | 2.0 | - | - |
| 15. Papain | - | - | 2.0 |
| 16. Hydroquinone | 2.0 | 2.0 | 2.0 |
| 17. Distilled water | To 100 | To 100 | To 100 |

### <Method for preparation>

1) A(ingredients 1∼9 and ingredient 17) and B (ingredients 10∼12) were heated to 75 °C.
2) A was slowly added to B, and the mixture was stirred at 7,500 rpm for 5 minutes to emulsify.
3) Ingredient 13 was added to the mixture, and stirred at 7,500 rpm for 5 minutes to emulsify.
4) Mixture of 3) was cooled in an ice bath to 25°C.
5) Ingredients 14 ~ 16 were added, and stirred at 2,500 rpm for mixing.
6) The product was left to stand at room temperature for 24 hours to obtain ointment.

### <Experimental Example 3> Measurement for skin absorption of hydroquinone

The amount of the hydroquinone absorbed into the skin was measured with the method described in Experimental Example 1 except that ointments prepared in the Example 3 and Comparative Example 5 were used. The results are shown in Table 3.

**<Table 3>**

| Skin absorption of the hydroquinone (µm/cm²/wt%) | | |
|---|---|---|
| Time(hour) | Example 3 | Comparative Example 5 |
| 0 | 0 | 0 |
| 4 | 18.66 | 3.67 |
| 8 | 37.05 | 6.99 |
| 24 | 109.43 | 19.47 |

Table 3 shows that when a stabilized protease is added to the composition, the skin absorption of hydroquinone is increased by about six times compared with a case that a stabilized protease is not added.

### <Experimental Example 4> Measurement for skin absorption of the kojic acid infused in the aqueous gel type cosmetic composition after 30 days of its preparation

The amount of the kojic acid absorbed into the skin was measured with the method described in Experimental Example 1 except that aqueous gel type cosmetic compositions of Example 1 and Comparative Example 2 after 30 days of their preparation were used. The results are shown in Table 4.

**<Table 4>**

| Skin absorption of the kojic acid (µm/cm²/wt%) | | |
|---|---|---|
| Time(hour) | Example 1 | Comparative Example 1 |
| 0 | 0 | 0 |
| 4 | 6.35 | 1.33 |
| 8 | 13.02 | 3.10 |
| 24 | 36.93 | 9.20 |

Table 4 shows that although papain is contained in the cosmetic composition, as shown in case of comparative example 2 that do not comprise stabilized papain, papain loses its activity after 30 days of its preparation by the effects of the other ingredients contained together, therefore papain no longer shows the skin absorption enhancement

### <Experimental Example 5> Measurement for skin absorption of the ascorbic acid infused in the water-in-oil cosmetic composition after 30 days of its preparation

The amount of the ascorbic acid absorbed into the skin was measured with the method described in Experimental Example 2 except that water-in-oil type cosmetic compositions of Example 2 and Comparative Example 4 after 30 days of their preparation were used. The results are shown in Table 5.

**<Table 5>**

| Skin absorption of the ascorbic acid (µm/cm²/wt%) | | |
|---|---|---|
| Time(hour) | Example 1 | Comparative Example 1 |
| 0 | 0 | 0 |
| 4 | 4.64 | 0.62 |
| 8 | 7.22 | 1.33 |
| 24 | 21.54 | 4.58 |

Table 5 shows that although papain is contained in the cosmetic composition, as shown in the case of comparative example 4 that does not comprise stabilized papain, papain loses its activity after 30 days of its preparation by the effects of the other ingredients contained together, therefore papain no longer shows the skin absorption enhancement.

### <Experimental Example 6> Measurement for skin absorption of hydroquinone confused in the ointment after 30 days of its preparation

The amount of the hydroquinone absorbed into the skin was measured with the method described in Experimental Example 3 except that ointments of Example 3 and Comparative Example 6 after 30 days of their preparation were used. The results are shown in Table 6.

**<Table 6>**

| Skin absorption of the hydroquinone (µm/cm²/wt%) | | |
|---|---|---|
| Time(hour) | Example 1 | Comparative Example 1 |
| 0 | 0 | 0 |
| 4 | 18.51 | 3.55 |
| 8 | 36.89 | 7.02 |
| 24 | 110.25 | 20.21 |

Table 6 shows that although papain is contained in the cosmetic composition, as shown in case of comparative example 6 that does not comprise stabilized papain, papain loses its activity after 30 days of its preparation by the effects of the other ingredients contained together, therefore papain no longer shows the skin absorption enhancement

### <Experimental Example 7> Observation of the skin injury

The external applications prepared in Examples 1 to 3 were applied on the skin of the guinea pig as described in the Experimental Example 1. When 24 hours had passed after applying, the skin was taken out from the Frantz permeation cell to be observed with naked eyes.

As the result of the observation, skin injury was not observed in the case of Examples 1 to 3, and it was found that stabilized protease do not cause skin injury but enhances skin absorption of the active agents.

### <Experimental Example 8> Skin stabilization

To examine the skin stabilization function of the protease, 300 ml of the external applications prepared in Examples 1 to 3 were applied on the same position of the back of nude mouse once a day for 30 days to observe the skin injuries such as swelling, erythema, et al. with naked eyes. However, no skin injury was observed.

The skin absorption enhancers of the present invention do not cause skin problems such as skin stimuli, skin injury, et al., but the do enhance the skin absorption of the components that have problems in permeation because of they have low affinity to the corneous layer or high molecular weight.

## Claims

1. Non therapeutic use of a protease stabilized by β-1,3-glucan branched with β-1,6 linkage as a skin absorption enhancing agent of an active agent of a cosmetic composition.

2. The use of claim 1, wherein the composition is a composition for external application.

3. The use of claim 1, wherein said active agent is selected from the group consisting of L-ascorbic acid, derivatives of L-ascorbic acid, vitamin B, kojic acid, kojic caffeine acid, kojic aminopropyl phosphate, hydroquinone, arbutin and water soluble natural.

4. The use of claim 1, wherein said β-1,3-glucan branched with β-1,6-linkage is schizophyllan, scleroglucan or lentinan.

5. The use of claim 1, wherein said proteases is selected from the group consisting of papain, trypsin, chymotrypsin, carboxypepidase and pepsin.

6. The use of claim 1, wherein said proteases is stabilized by the method comprising the steps of :
(1) reacting β-1,3-glucan branched with β-1,6-linkage and periodates to transform the β-1,6-linkage of the glucan into aldehyde ;
(2) removing unreacted periodates from the reaction solution of step (1) ;
(3) adding a protease to the reaction solution of step (2) in an amount of 0.00001 ∼ 10.0% weight ;
(4) adding a reducing agent to the reaction solution of step (3) in an amount of 0.0001 ∼ 1.0% by weight ; and
(5) washing the product of step (4).

## Patentansprüche

1. Nicht-therapeutische Verwendung einer Protease, die durch β-1,3-Glucan, das mit β-1,6-Verknüpfung verzweigt ist, stabilisiert ist, als ein Hautabsorptions-Verstärkungsmittel eines aktiven Mittels einer kosmetischen Zusammensetzung.

2. Verwendung gemäß Anspruch 1, wobei die Zusammensetzung eine Zusammensetzung für äußerliche Anwendung ist.

3. Verwendung gemäß Anspruch 1, wobei das aktive Mittel ausgewählt ist aus der Gruppe bestehend aus L-Ascorbinsäure, Derivaten von L-Ascorbinsäure, Vitamin B, Kojisäure, Koji-Koffeinsäure, Koji-Aminopropylphosphat, Hydrochinon, Arbutin und wasserlöslichen Naturstoffen.

4. Verwendung gemäß Anspruch 1, wobei das β-1,3-Glucan, das mit β-1,6-Verknüpfung verzweigt ist, Schizophyllan, Scleroglucan oder Lentinan ist.

5. Verwendung gemäß Anspruch 1, wobei die Proteasen ausgewählt sind aus der Gruppe bestehend aus Papain, Trypsin, Chymotrypsin, Carboxypepidase und Pepsin.

6. Verwendung gemäß Anspruch 1, wobei die Proteasen durch das Verfahren stabilisiert sind, das die Schritte umfasst:
(1) Umsetzen von β-1,3-Glucan, das mit β-1,6-Verknüpfung verzweigt ist und Periodaten, um die β-1,6-Verknüpfung des Glucans in Aldehyd zu überführen;
(2) Entfernen von nicht umgesetzten Periodaten aus der Reaktionslösung aus Schritt (1);
(3) Hinzufügen einer Protease zu der Reaktionslösung aus Schritt (2) in einer Menge von 0,00001 - 10,0 Gewichts-%;
(4) Hinzufügen eines Reduktionsmittels zu der Reaktionslösung aus Schritt (3) in einer Menge von 0,0001 - 1,0 Gewichts-%; und
(5) Waschen des Produkts aus Schritt (4).

## Revendications

1. Utilisation non thérapeutique d'une protéase stabilisée par le β-1,3-glucane ramifié avec une liaison β-1,6, en tant qu'agent facilitant l'absorption par voie cutanée d'un principe actif d'une composition cosmétique.

2. Utilisation selon la revendication 1, dans laquelle la composition est une composition pour application externe.

3. Utilisation selon la revendication 1, dans laquelle ledit principe actif est choisi dans le groupe constitué de l'acide L-ascorbique, des dérivés de l'acide L-ascorbique, de la vitamine B, de l'acide kojique, de l'acide caféine kojique, de l'aminopropylphosphate kojique, de l'hydroquinone, de l'arbutine et de composés naturels hydrosolubles.

4. Utilisation selon la revendication 1, dans laquelle ledit β-1,3-glucane ramifié avec une liaison β-1,6 est la schizophyllane, le scléroglycane ou le lentinane.

5. Utilisation selon la revendication 1, dans laquelle ladite protéase est choisie dans le groupe constitué de la papaïne, de la trypsine, de la chymotrypsine, de la carboxypeptidase et de la pepsine.

6. Utilisation selon la revendication 1, dans laquelle ladite protéase est stabilisée par le procédé comprenant les étapes consistant à :
(1) faire réagir le β-1,3-glucane ramifié avec une liaison β-1,6 avec des periodates pour transformer la liaison β-1,6 du glucane en aldéhyde ;
(2) éliminer les periodates n'ayant pas réagi de la solution réactionnelle de l'étape (1) ;
(3) ajouter une protéase à la solution réactionnelle de l'étape (2) en une quantité allant de 0,00001% à 10,0% en poids ;
(4) ajouter un agent réducteur à la solution réactionnelle de l'étape (3) dans une quantité allant de 0,0001% à 1,0% en poids ; et
(5) laver le produit de l'étape (4).
